# EUROPEAN PATENT SPECIFICATION

(11) **EP 0 470 778 B1**
(45) Date of publication and mention of the grant of the patent: **25.05.1994**
(21) Application number: 91307126.2
(22) Date of filing: 02.08.1991
(51) Int. Cl.: A61F 2/36

(54) **Total hip replacement femoral component**
Femurprothese zum Totalersatz des Hüftgelenks
Prothèse fémorale pour remplacement total de l'articulation totale de la hanche

(30) Priority: 08.08.1990 GB 9017402
(43) Date of publication of application: 12.02.1992
(73) Proprietor: HOWMEDICA INTERNATIONAL INC., Shannon Co. Clare (IE)
(72) Inventor: Ling, Robin Sidney, Dittisham, Dartmouth TQ6 0HB (GB); Lawes, Peter, Maidenhead, Berkshire SL6 4DB (GB)
(74) Representative: Bridge-Butler, Alan James

(56) References cited:
- FR-A- 2 304 324
- FR-A- 2 361 861
- US-A- 4 123 806
- US-A- 4 846 841

## Description

This invention relates to a total hip replacement femoral component.

Total hip replacements conventionally use a long intramedullary stem passing the marrow cavity of the bone. These are very successful but when they fail the long stem can create considerable damage inside the bone. It can cause a fracture through the bone around the level of the tip of the stem. The implant can wobble about inside the bone causing the intramedullary cavity to become larger and larger. Revision of failures is always difficult for a surgeon.

Surgeons have always called for a more conservative device. The Smith-Peterson cups of the 1920's and 1930's were merely free floating substantially hemispherical shells placed between the top of the femur and the acetabulum. Apart from paring away the cartilage there was no bone removal. They were not very successful and the constant movement of bone against metal as the joint moved eventually caused severe bony erosion. In the mid-1940's Judet in France designed a prosthesis whereby the majority of the femoral head was removed and a replacement device was fitted with a peg or nail which passed a short way down the femoral neck. These lasted a little longer than the Smith-Peterson cups but not much. Small movement of the device against the bone caused friction of the bone and the bending loads on the peg often caused them to break out underneath the bony femoral neck. In the mid-1970′s double cup arthroplasty was tried. There were several designs: Wagner in Germany, an Italian Group, Imperial College and London Hospital (ICLH) and the Tharies design from Amstutz in California. These all removed a fair proportion of the femoral bearing surface turning it down to a cylindrical form or hemispherical form and a metal shell was then fixed with bone cement unto the remaining bony peg. The acetabular cup was quite conventional. Unlike normal total hips however which have standard femoral head sizes of 22 mm, 26 mm, 28 mm, 32 mm, these double cup arthroplasties have to have large bearing surface diameters closer to the original hip, typically 40 mm or 41 mm for a small size, 45 mm or 46 mm for a medium size and 50 mm or 51 mm for a large size. These latter double cup designs commonly failed either by a crack progressing through or adjacent to the bone cement between the prosthetic femoral shell and the bone or by a fracture of the bone across from one side of the prosthetic femoral component rim to the other.

US Patent 4 123 806 discloses a total hip replacement femoral component having an outer part-spherical surface shaped to conform to an acetabular socket and having an internal bore adapted to receive a femoral head which has been shaped and dimensioned to provide a spigot to enter it and which can be locked in place by a cement mantle. This type of construction was commercialised as the "THARIES HIP". There is no special provision for the cement to creep in this construction because there is nowhere provided for it to flow to unless it oozes out around the peripheral opening between the femoral head and the rim of the bore in the component. In practice it was found that the stress in the cement can be highest around the rim causing cracking and there is no provision for sliding and relocking. Alternatively, if the stress in the cement in this construction is close to the pole of the prosthesis shell then there is, by design, nowhere for the cement to creep in order to relieve the stress. If the stress is high enough this causes cracking and the cracks progress and the device becomes loose. This is exactly what happened clinically with the THARIES HIP and similar designs as sold commercially.

The present invention is intended to provide a more successful surface replacement of the femoral side of a total hip because the absence of the stem is undoubtedly more conservative and is preferred over the stemmed variety by a great many surgeons.

According to the present invention a total hip replacement femoral component having an outer part spherical surface shaped to conform to an acetabular socket and having an internal bore adapted to receive a femoral head which has been shaped and dimensioned to provide a spigot to enter it and be locked in place by a cement mantle, characterised by means for preventing cement reaching the bottom of the bore and for creating a space between the bottom end of said bore and the end of said spigot to allow said component to move further over said cement mantle onto said spigot and re-lock should the cement creep or there be any movement in the bone after assembly.

The bone can be prepared to provide a cylindrical or slightly tapering spigot.

Preferably the internal bore is inwardly tapered. Thus the taper can be co-axial with the femoral neck but there may be advantages in orientating the axis of the taper slightly more vertical when in position so that it is closer to the average force vector acting on the femoral head during human activity.

With this tapered bore the interface between the bone cement and the bone is protected against shear and damage and any movement preferably takes place between the bone cement and the prosthesis.

The bore may be arranged to be slightly oval or it can be multi-faceted or it can be provided with grooves and/or ridges which extend longitudinally thereof to prevent rotation relative to said spigot.

Alternatively the bore may be circular and there may be provision for causing the femoral component to rotate slightly if there is any inward movement of the bone into the component.

The bore may conveniently have a smooth polished surface to ensure that any movement which takes place is preferably between the bone cement or the outer surface of the space creator and the prosthesis and not between the bone cement and the bone itself.

A space creator may be included for preventing cement reaching the bottom of said bore, said space creator causing a space into which said spigot can move after assembly if the cement creeps or if there is any movement in the bore.

This space creator may comprise a cap which is dimensioned to fit in said bore towards the inner end thereof and the external side of which is engaged by said spigot when located therein, the cap can be a flat disc or it can be cup-shaped having side walls within which said spigot is located when in position.

In another preferred construction the walls of the cup-shaped cap extend outwardly to cover all the internal wall of the bore surrounding the spigot when in position.

The space creator can be pre-fitted either by the manufacturer or by the surgeon inside the bore and is preferably made of a material similar to cement, for example polymethylmethacrylate to make it fully compatible with the bone cement.

The space which is left can be typically 3 mm or 4 mm inside the femoral head. With this arrangement the bone cement can be applied to the bone or to the prosthetic femoral head immediately before the implant is fitted.

In another arrangement the space creator can be in the form of a collar which fits over the spigot towards its proximal end but with this arrangement a surgeon must be careful to apply the cement around the exposed portion of bore, not over the space creator.

The space creator acts to allow the spigot to sink further into the bore without its proximal end reaching the bottom end of the bore and preventing this internal movement.

The bore may conveniently have a polished surface to ensure that any movement which takes place is preferably between the bone cement or the outer surface of the space creator and the prosthesis and not between the bone cement and the bone itself.

Any rotation preventing feature, such as bore ovality or multi-facets or grooves must extend lengthwise, or substantially lengthwise and may include slight spiralling, no circumferential grooves or ridges can be permitted which will resist re-engagement of the tapered spigot with the taper bore.

In the arrangement to allow the femoral component to rotate means can be provided on the space creator itself or by causing the cement to take up appropriate shapes and dimensions.

The invention also includes a total hip prosthesis incorporating a femoral component as set forth above.

The invention can be performed in many ways and two embodiments will now be described by way of example and with reference to the accompanying drawings in which :
Figure 1 is a diagrammatic cross-section through a femur provided with the femoral component according to the invention;
Figure 2 is a similar view of an alternative construction; and,
Figures 3, 4, 5 and 6 are similar views of further constructions.

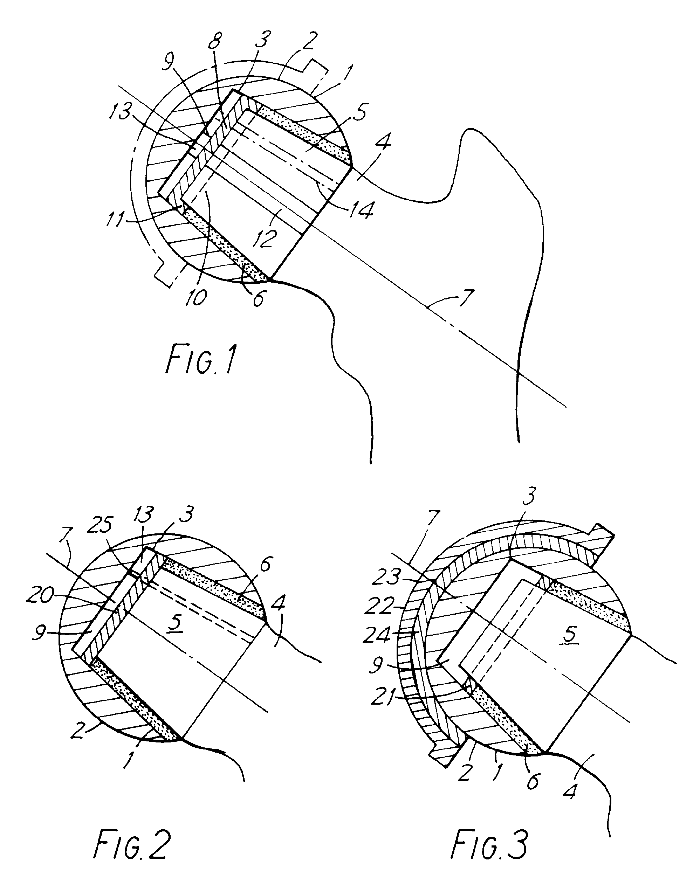

As shown in Figure 1 of the drawings the total hip replacement femoral component indicated by reference numeral 1 has an outer surface 2 in the usual ball shape to conform to an acetabular socket indicated by chain lines. An internal bore 3 is provided which is inwardly tapered at an angle of about 1° to 20° and in the arrangement shown is substantially circular. The component is made from for example a stainless steel and the internal surface of the bore 3 is highly polished. If desired the taper could be parabolic or a truncated parabola.

The femoral head 4 on which the femoral component is to be used is shaped and dimensioned to provide a substantially circular tapered spigot 5, the angle of the taper being similar to the angle of taper of the bore 3 and being dimensioned so that it will lock into position when held in place by cement 6. The taper of the spigot 5 is coaxial with the femoral neck, the general axis of which is indicated by the broken line 7. There may however be an advantage in orientating the axis of the taper closer to the vertical so that it is closer to the average force vector acting on the femoral head during human activity.

A space creator 8 is provided which is shaped and dimensioned to fit closely within the bore 3 towards its bottom end but leaving a space 9 which may be typically 3 mm or 4 mm deep. It will be seen that this space creator 8 is provided as a cup-shaped cap for the spigot when it is in position and is dimensioned so that the side walls 11 of the cup locate the proximal end 10 of the spigot when it is in position. This space creator 8 can be pre-fitted into the bore 3 either by the manufacturer or the surgeon prior to use and is made from polymethylmethacrylate so that it is fully compatible with the bone cement which will be of the same material.

If desired the space creator could be provided as a substantially flat disc 20 as shown in Figure 2, in which the same reference numerals are used to indicate similar parts.

Also shown in Figure 2 is a groove 25 which can be included on the wall of the bore 3 at the interface between the component 2 and the space creator to allow air to escape. Although only one groove 25 is shown two or more such grooves could be provided. Grooves of this type can also be included in the construction shown in the other Figures but they have been omitted for clarity.

To prevent the femoral component rotating on the spigot 5 the bore 3 and the spigot 5 can be made slightly oval or the spigot can be provided with a flat facet 12 which is aligned with a similar flat facet 13 in the bore. With this arrangement the cement 6 fills in a space between the facets 12 and 13 and thus provides what is in effect a flat locking element.

In another alternative construction a groove indicated by broken lines 14 can be provided on the component so that cement in the space between the component and the bone fills the groove and acts as a locking key. These locking devices could be used in any of the other constructions shown but are omitted for clarity. In all the arrangements, however, these anti-rotation features extend lengthwise of the bore 3 and may be straight or slightly curved. With this type of arrangement no spiral features or circumferential grooves or ridges are permitted.

In order to locate the femoral component on the bone the femoral head is first shaped to provide the tapered spigot 5 and the bore 3 or the surface of the spigot 5 is covered with suitable cement. The femoral component 1 is now pushed into the position shown in the drawing, the cement mantle and the taper acting to hold the component in place and the appropriate features preventing rotation.

The interface between the bone cement and the bone is protected against shear and damage and the interface between the walls of the bore 3 and the cement is such that should there be any creep in the cement or movement in the bone this is taken up by the component moving further over the spigot 5, the polished surface of the bore 3 allowing this movement to take place and the taper enabling the parts to lock together again.

The space creator prevents cement entering the space 9 so that there is space within the bore for the spigot to move further inwardly, the space creator moving with the spigot.

Thus, should any loosening tend to occur the assembly will re-lock.

In an alternative arrangement as shown in Figure 3, in which the same reference numerals are again used, the space creator is provided merely by a collar 21 of, for example, polymethylmethacrylate and which will be slipped over the proximal end of the spigot and which will act as a seal to prevent cement entering the portion of the bore beyond the proximal end of the spigot thus creating the necessary space. With this arrangement however it is necessary for the surgeon to apply the cement only to the surface of the spigot beyond the space creating collar so that when the component is pushed into place cement cannot enter the space. Figure 3 also shows an acetabular socket 22 comprising an outer shell 23 and a bearing liner 24, which can be used with the replacement femoral component of the invention, makes up a total prosthesis.

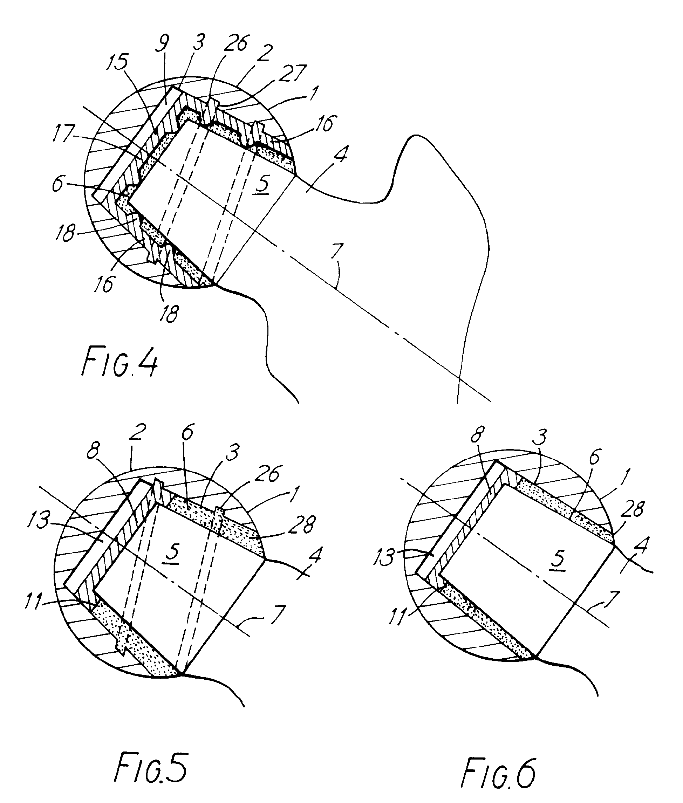

In another construction according to the invention and as shown in Figure 4 the same reference numerals are again used to indicate similar parts. In this construction however a space creator 15 is provided which is generally cup-shaped and has side walls 16 which extend from a base 17 outwardly for the remaining full length of the socket 3. The side walls are a close fit in the socket 3 and the cup is made from polymethylmethacrylate so that it is fully compatible with the bone cement which is used and, in fact, provides what is in effect a preformed cement mantle. The space creator with its side walls is made prior to fitting and is not moulded within the socket. Thus it can move in the socket to absorb relative movement between the bone and the femoral component.

If desired protuberances 18 can be formed integral with the preformed space creator and these act to locate the spigot 5 when it is inserted. The bone cement 6 acts to secure the spigot 5 to the mantle provided by the void creator and becomes effectively integral with it. Thus any relative movement between the parts tends to be absorbed by movement of the preformed cement mantle provided by the space creator and the wall of the bore 3.

Figure 4 also shows a construction in which the femoral component can rotate slightly rather than having the anti-rotation features described with regard to Figure 1. Thus, the inner surface of the bore 3 is provided with a spiral groove 26 and the outer surface of the side wall 16 of the space creator 15 are provided with a spiral ridge to co-operate with the groove. Any inward movement of the bone and the space creator into the bore 3 of the femoral component therefore creates a slight relative rotation.

If it is desired to allow this type of rotation between the femoral component and the bone then the anti-rotation means shown in Figure 1 can be replaced appropriately in any of the constructions shown.

Figure 5 shows another construction in which relative rotation is again allowed but in this case the configuration of the space creator is similar to that shown in Figure 1, that is the side walls do not extend throughout the length of the bore 3 of the component. As will be seen from Figure 5 the spiral groove 26 is again provided but with this arrangement the groove is filled by the cement 6 which, when it has set, provides what is in effect ridges extending into the grooves 26 so that the movement is created.

Figure 5 also shows a construction in which the wall of the internal bore 3, although being inwardly tapered, is not parallel with the wall of the tapered spigot 5. It will be seen that the angle of the wall of the bore 3 is less than the angle of the wall of the spigot 5 so that the thickness of the cement 6 at the outer end 28 of the bore 3 is thicker than at the inner end.

Figure 6 shows another construction in which the taper angles are dissimilar and which are arranged so that the thickness of the cement mantle is shallower at the open end 28 than at the inner end. Such an arrangement can also be achieved by making the internal bore 3 of parabolic form. Such constructions can again be provided with anti-rotation features or with rotation features as desired.

The invention also includes a total hip prosthesis incorporating a femoral component as described above. Thus the total assembly could provide an artificial hip cup together with the femoral component of the kind set forth.

## Claims

1. A total hip replacement femoral component (1) having an outer part spherical surface (2) shaped to conform to an acetabular socket and having an internal bore (3) adapted to receive a femoral head (4) which has been shaped and dimensioned to provide a spigot (5) to enter it and be locked in place by a cement mantle (6), characterised by means (8) for preventing cement (6) reaching the bottom of the bore (3) and for creating a space (9) between the bottom end of said bore (3) and the end (10) of said spigot (5) to allow said component (1) to move further over said cement mantle (6) onto said spigot (5) and re-lock should the cement (6) creep or there be any movement in the bone after assembly.

2. A total hip replacement femoral component as claimed in claim 1 characterised in that said bore (3) is substantially circular.

3. A total hip replacement femoral component as claimed in claim 2 characterised in that said bore (3) is slightly oval.

4. A total hip replacement femoral component as claimed in claim 2 or claim 3 characterised in that said bore (3) is multi-faceted, grooved and/or provided with ridges which extend longitudinally thereof to prevent rotation relative to said spigot (5).

5. A total hip replacement femoral component as claimed in any one of claims 1 to 4 characterised in that said bore (3) has a smooth polished surface.

6. A total hip replacement femoral component as claimed in any one of claims 1 to 5 characterised in that the means for creating said space (9) comprise a space creator (8) which prevents cement (16) reaching the bottom end of said bore (3) and creates a space (9) into which said spigot (5) can move after assembly if the cement (16) creeps or there is any movement in the bore (3).

7. A total hip replacement femoral component as claimed in claim 6 characterised in that said space creator (8) comprises a cap which is dimensioned to fit in said bore (3) towards the bottom end thereof and the external side of which is engaged by said spigot (5) when located in said bore (3).

8. A total hip replacement femoral component as claimed in claim 7 characterised in that said cap is a flat disc (20).

9. A total hip replacement femoral component as claimed in claim 7 characterised in that said cap is cup-shaped having side walls (11) within which said spigot (5) is located when in position.

10. A total hip replacement femoral component as claimed in claim 9 characterised in that the walls (11) of the cup-shaped cap (8) extend outwardly to cover all the wall of the bore (3) surrounding the spigot (5) when in position.

11. A total hip replacement femoral component as claimed in claim 6 characterised in that said space creator is in the form of a collar (21) which fits over the spigot (5) towards its proximal end (10).

12. A total hip replacement femoral component as claimed in any one of claims 6 to 11 characterised in that said space creator (8, 20, 21) is made of material similar to cement.

13. A total hip replacement femoral component as claimed in claim 12 characterised in that said space creator (8, 20, 21) is made from polymethylmethacrylate.

14. A total hip prosthesis incorporating a femoral component as claimed in any one of the preceding claims.

## Patentansprüche

1. Femurprothese (1) für einen Hüftgelenk-Totalersatz, mit einer äußeren teilsphärischen Oberfläche (2), die so geformt ist, daß sie sich an eine Hüftpfanne anpaßt, und mit einer inneren Bohrung (3), die zur Aufnahme eines Femurkopfes (4) angepaßt ist, welcher so geformt und bemessen worden ist, daß ein in sie eintretender und mittels eines Zementmantels (6) in der vorgesehenen Lage festzuhaltender Zapfen (5) geschaffen wird, gekennzeichnet durch Mittel (8), um zu verhindern, daß Zement (6) den Boden der Bohrung (3) erreicht, und um zwischen dem bodenseitigen Ende der besagten Bohrung (3) und dem Ende (10) des besagten Zapfens (5) einen Raum (9) zu schaffen, so daß sich die besagte Prothese (1) weiter über den besagten Zementmantel (6) auf den besagten Zapfen (5) bewegen und sich erneut festgehalten werden kann, falls der Zement (6) kriechen sollte oder falls nach einem Zusammenbau irgendeine Bewegung im Knochen stattfinden sollte.

2. Femurprothese für einen Hüftgelenk-Totalersatz nach Anspruch 1, dadurch gekennzeichnet, daß die besagte Bohrung (3) im wesentlichen rund ist.

3. Femurprothese für einen Hüftgelenk-Totalersatz nach Anspruch 2, dadurch gekennzeichnet, daß die besagte Bohrung (3) geringfügig oval ist.

4. Femurprothese für einen Hüftgelenk-Totalersatz nach Anspruch 2 oder Anspruch 3, dadurch gekennzeichnet, daß die besagte Bohrung (3) mehrfach facettiert, mit Nuten und/oder mit Graten versehen ist, welche sich in ihrer Längsrichtung erstrecken, um eine Drehung relativ zu dem besagten Zapfen (5) zu verhindern.

5. Femurprothese für einen Hüftgelenk-Totalersatz nach einem beliebigen der Ansprüche 1 bis 4, dadurch gekennzeichnet, daß die besagte Bohrung (3) eine glatte polierte Oberfläche besitzt.

6. Femurprothese für einen Hüftgelenk-Totalersatz nach einem beliebigen der Ansprüche 1 bis 5, dadurch gekennzeichnet, daß die Mittel zum Schaffen des besagten Raums (9) eine raumschaffende Vorrichtung (8) umfassen, welche verhindert, daß Zement (16) das bodenseitige Ende der besagten Bohrung (3) erreicht und einen Raum (9) schafft, in welchen sich der besagte Zapfen (5) nach einem Zusammenbau bewegen kann, falls der Zement (16) kriecht oder irgendeine Bewegung in der Bohrung (3) erfolgt.

7. Femurprothese für einen Hüftgelenk-Totalersatz nach Anspruch 6, dadurch gekennzeichnet, daß die besagte raumschaffende Vorrichtung (8) eine Kappe umfaßt, die so bemessen ist, daß sie auf das bodenseitige Ende der besagten Bohrung (3) zu in diese hineinpaßt, und mit deren äußerer Seite der besagte Zapfen (5) im Eingriff steht, wenn er in der besagten Bohrung (3) fixiert ist.

8. Femurprothese für einen Hüftgelenk-Totalersatz nach Anspruch 7, dadurch gekennzeichnet, daß die besagte Kappe eine flache Scheibe (20) ist.

9. Femurprothese für einen Hüftgelenk-Totalersatz nach Anspruch 7, dadurch gekennzeichnet, daß die besagte Kappe napfförmig ist und Seitenwände (11) aufweist, innerhalb derer der besagte Zapfen (5) fixiert ist, wenn er sich in der vorgesehenen Position befindet.

10. Femurprothese für einen Hüftgelenk-Totalersatz nach Anspruch 9, dadurch gekennzeichnet, daß sich die Wände (11) der napfförmigen Kappe (8) auswärts erstrecken, so daß sie die gesamte, den in der vorgesehenen Position befindlichen Zapfen (5) umgebende Wand der Bohrung (3) bedecken.

11. Femurprothese für einen Hüftgelenk-Totalersatz nach Anspruch 6, dadurch gekennzeichnet, daß die besagte raumschaffende Vorrichtung in Form eines Kragens (21) vorliegt, der auf das proximale Ende (10) des Zapfens (5) zu über diesen paßt.

12. Femurprothese für einen Hüftgelenk-Totalersatz nach einem beliebigen der Ansprüche 6 bis 11, dadurch gekennzeichnet, daß die besagte raumschaffende Vorrichtung (8, 20, 21) aus einem zementähnlichen Material hergestellt ist.

13. Femurprothese für einen Hüftgelenk-Totalersatz nach Anspruch 12, dadurch gekennzeichnet, daß die besagte raumschaffende Vorrichtung (8, 20, 21) aus Polymethylmethacrylat hergestellt ist.

14. Hüftgelenk-Totalprothese beinhaltend eine Femurprothese nach einem beliebigen der vorangehenden Ansprüche.

## Revendications

1. Composant fémoral (1) de prothèse totale de hanche comprenant un élément externe à surface sphérique (2) formé de manière à se conformer à une cavité acétabulaire et comprenant un alésage interne (3) adapté à recevoir une tête fémorale (4) qui a été conformée et dimensionnée de manière à constituer une queue de fixation (5) pour y pénétrer et être bloquée en place par une enveloppe en ciment (6), caractérisé par des moyens (8) pour éviter que le ciment (6) atteigne le fond de l'alésage (3) et pour créer un espace (9) entre l'extrémité de fond dudit alésage (3) et l'extrémité (10) de ladite queue de fixation (5) pour permettre audit composant (1) de continuer de se déplacer sur ladite enveloppe en ciment (6) sur ladite queue de fixation (5) et de se rebloquer dans le cas où le ciment (6) flue ou quand il y a un mouvement quelconque dans l'os après assemblage.

2. Composant fémoral de prothèse totale de hanche selon la revendication 1, caractérisé en ce que l'alésage (3) est sensiblement circulaire.

3. Composant fémoral de prothèse totale de hanche selon la revendication 2, caractérisé en ce que ledit alésage (3) est légèrement ovale.

4. Composant fémoral de prothèse totale de hanche selon la revendication 2 ou la revendication 3, caractérisé en ce que ledit alésage (3) est à facettes multiples, à gorge et/ou muni de crêtes qui s'étendent longitudinalement sur lui pour éviter une rotation relative par rapport à ladite queue de fixation (5).

5. Composant fémoral de prothèse totale de hanche selon l'une quelconque des revendications 1 à 4, caractérisé en ce que ledit alésage (3) présente une surface polie et lisse.

6. Composant fémoral de prothèse totale de hanche selon l'une quelconque des revendications 1 à 5, caractérisé en ce que les moyens pour créer ledit espace (9) comprennent un créateur d'espace (8) qui empêche le ciment (16) d'atteindre l'extrémité de fond dudit alésage (3) et crée un espace (9) dans lequel ladite queue de fixation (5) peut se déplacer après assemblage si le ciment (16) flue ou s'il y a un mouvement quelconque dans l'alésage (3).

7. Composant fémoral de prothèse totale de hanche selon la revendication 6, caractérisé en ce que ledit créateur d'espace (8) comprend un capuchon qui est dimensionné pour se disposer dans ledit alésage (3) en direction de son extrémité de fond et dont le côté externe est en engagement avec ladite queue de fixation (5) quand elle est disposée dans ledit alésage (3).

8. Composant fémoral de prothèse totale de hanche selon la revendication 7, caractérisé en ce que ledit capuchon est un disque plat (20).

9. Composant fémoral de prothèse totale de hanche selon la revendication 7, caractérisé en ce que ledit capuchon est en forme de cuvette comportant des parois latérales (11) à l'intérieur desquelles ladite queue de fixation (5) est disposée quand il est en position.

10. Composant fémoral de prothèse totale de hanche selon la revendication 9, caractérisé en ce que les parois (11) du capuchon en forme de cuvette (8) s'étendent vers l'extérieur pour recouvrir la totalité de la paroi de l'alésage (3) qui entoure la queue de fixation (5) quand il est en position.

11. Composant fémoral de prothèse totale de hanche selon la revendication 6, caractérisé en ce que ledit créateur d'espace a la forme d'un collier (21) qui se dispose sur la queue de fixation (5) en direction de son extrémité proximale (10).

12. Composant fémoral de prothèse totale de hanche selon l'une quelconque des revendications 6 à 11, caractérisé en ce que ledit créateur d'espace (8, 20, 21) est réalisé en un matériau similaire au ciment.

13. Composant fémoral de prothèse totale de hanche selon la revendication 12, caractérisé en ce que ledit créateur d'espace (8, 20, 21) est réalisé en polyméthacrylate de méthyle.

14. Prothèse totale de hanche incorporant un composant fémoral selon l'une quelconque des revendications précédentes.
